# EUROPEAN PATENT APPLICATION

(11) **EP 2 737 902 A1**
(43) Date of publication of application: **04.06.2014**
(21) Application number: 12194594.3
(22) Date of filing: 28.11.2012
(51) Int. Cl.: A61K 31/426, A61K 45/00, A61K 31/722, A61P 1/02, A61P 17/02, A61P 29/00

(54) **Compositions comprising cimetidine and n,o-carboxymethyl chitosan for the prevention and therapy of pathologies of the skin and the mucous membranes and in the odontostomatological field**

(71) Applicant: Zarzatech, Inc., Cambridge, MA 02142 (US)
(72) Inventor: Divney, Sonia, 1091 Grandvaux (CH); Di Schiena, Michele G., 20087 ROBECCO SUL NAVIGLIO (MI) (IT)
(74) Representative: Sweetinburgh, Mark Roger

(57) **Abstract**

Topical compositions comprising an H2 receptor antagonist and cimetidine and a chitosan soluble derivative in admixture with suitable carriers and excipients.

## Description

The present invention relates to compositions comprising an H2 receptor antagonist and a chitosan soluble derivative for the prevention and therapy of pathologies of the mucous membranes.

In particular, the compositions of the invention are useful for the prevention and therapy of odontostomatological pathologies characterized by inflammation and pain, in particular for the prevention and therapy of pathologies of the periodontium.

The compositions of the invention are intended for the human and veterinary use.

### TECHNOLOGICAL BACKGROUND

Cimetidine has been used for some time in gastroenterology in the treatment of benign gastric and duodenal ulcers, reflux esophagitis, Zollinger-Ellison sindrome, sistemic mastocytosis and multiple endocrine adenomas. Cimetidine is an antagonist of the H2 receptors of the gastric mucous membranes, where it reduces the acid secretion induced by histamine.

Cimetidine is also used in the treatment of pathologies of the oral cavity, in particular of periodontium pathologies characterized by inflammation and pain (US 2008/0045575, US 5,294,433 and 5,364,616).

Cimetidine is used in topical pharmaceutical forms, preferably in aqueous based ones, for example mouth-washes, for the treatment of pathologies of the oral cavity, in particular of periodontium pathologies.

Topical administration of Cimetidine involves however the problem that the medicament exerts a reduced action, which is further negatively affected by its water solubility, also following the detergent action of the saliva.

### DISCLOSURE OF THE INVENTION

It has now been found that the addition of a chitosan soluble derivative to topical compositions of histamine H2 receptor antagonists is advantageous in that it gives the compositions surprising mucoadhesive properties.

Anti-H2 antagonists such as Cimetidine, Ranitidine, Famotidine, Nizatidine and the like, in addition to the therapy of gastric and duodenal ulcers and other gastro-intestinal diseases characterized by acid hypersecretion, can also be used for inflammatory diseases of the mucous membranes.

From this point of view, Cimetidine is at present the most studied molecule.

A chitosan soluble derivative contains carboxy or sulfonic, preferably carboxy, ionizable groups. An example of chitosan soluble derivative according to the invention is N,O-Carboxymethyl chitosan, whose preparation is reported in U.S 4,619,995.

N,O-Carboxymethyl chitosan, easily commercially available (for example from HEPPE MEDICAL CHITOSAN GmbH under the trade mark Chitoceuticals®) has useful properties in the odontostomatological field, in particular hydrating e film-forming actions, protective action of the mucous membranes against pathogenic agents and buffering action on the oral cavity physiological pH.

The invention therefore relates therefore to topical compositions comprising an H2 receptor antagonist, preferably cimetidine, and a chitosan soluble derivative, preferably N,O-Carboxymethyl chitosan, in admixture with suitable carriers and excipients.

The concentration of the chitosan derivative, in particular N,O-Carboxymethyl chitosan, ranges between 0.001% p/p and 10% p/p, preferably between 0.01 % p/p and 5% p/p.

The concentration of the H2 antagonist, in particular cimetidine, ranges between 0.0 1 % p/p and 10% p/p, preferably between 0.1% p/p and 5% p/p.

The excipients or carriers used in the compositions of the invention should of course be compatible with Cimetidine or the other H2 receptor antagonists and with the chitosan derivative. Said conventional excipients and carriers are described in Handbook of Pharmaceutical Excipients, 6th Edition, Pharmaceutical Press.

The compositions of the invention can be prepared according to known methods, described for example in Remington, The Science and Practice of Pharmacy, 20th Edition, typically in the form of mouth-washes, gel, spray, foams, emulsions, dentifrices, for use in the treatment of affections of the oral mucous membranes (periodontitis and the like), and in the form of gel, ointments, creams, powders, patches and transdermal forms for use in the treatment of lesions and wounds of the skin or of rectal and vaginal mucous membranes.

The compositions of the invention can further comprise other active principles useful for the topical treatment of oral mucous membranes, described for example in Martindale, The Complete Drug Reference, 34 th Edition. Examples of said further active principles comprise disinfectants of the oral cavity such as Propolis, chlorhexidine, benzalkonium, cetylpyridinium, Triclosan, silver and derivatives; steroidal or non steroidal anti-inflammatories such as Cortisone emisuccinate, Diclofenac, Ibuprofen, Ketoprofen; wound-healing agents such as Aloe vera, allantoin and derivatives, Liquorice and derivatives; analgesics such as Lidocaine and Benzydamine; antimicrobials, antihistaminics.

The mucoadhesive action of the compositions is promoted and further increased by the presence of water: water based mouth-washes are therefore particularly preferred. The physiological water present in the oral cavity also ensures an increase in mucoadhesivity even to compositions with very low - if any - water content.

Mucoadhesivity allows for Cimetidine or the other antagonists H2 to remain for a longer time on mucous membranes, in particular on the oral mucous membranes, thus enhancing the penetration of the medicament with evident, advantageous therapeutic benefits.

The compositions of the invention have the following further advantageous aspects:
a) stability in a wide pH range, between 4 and 8;
b) stability to thermal changes in a wide range of temperatures, in particular between -10°C and +40°C;
c) stability to a number of ingredients (active principles and excipients) conventionally used in the topical preparations;
d) stability to dilution, even high, with water.

The examples reported hereinbelow further illustrate the invention.

Percentages are expressed in parts by weight.

### Example 1 - mouth-wash

| | |
|---|---|
| Cimetidine | 0.5% |
| N,O-Carboxymethyl chitosan | 0.50% |
| 70% Sorbitol FU | 47.67% |
| Sodium benzoate | 1.00% |
| Xylitol | 0.60% |
| Disodium EDTA | 0.013% |
| Flavors | 0.30% |
| Dye E 124 | q.s. |
| Citric acid | q.s. to pH 6.8-7.2 |
| Osmosized water | q.s. to 100% |

### Example 2 - oral gel

| | |
|---|---|
| Cimetidine | 0.60% |
| N,O-Carboxymethyl chitosan | 0.70% |
| Sodium alginate | 1.20% |
| 70% Sorbitol FU | 5.00% |
| Preservative | q.s. |
| Flavors | q.s. |
| Dye E 124 | q.s. |
| Osmosized water | q.s. to 100% |

### Example 3 - oral spray

| | |
|---|---|
| Cimetidine | 0.60% |
| N,O-Carboxymethyl chitosan | 0.40% |
| Allantoin | 0.20% |
| Preservative | q.s. |
| Flavors | q.s. |
| Osmosized water | q.s. to 100% |

## Claims

1. Topical compositions comprising an H2 receptor antagonist and a chitosan soluble derivative in admixture with suitable carriers and excipients.

2. Compositions according to claim 1 wherein the H2 receptor antagonist is selected from Cimetidine, Ranitidine, Famotidine, Nizatidine, preferably Cimetidine.

3. Compositions according to claim 1 or 2 wherein the chitosan soluble derivative is N,O-Carboxymethyl chitosan.

4. Compositions according to one or more of claims 1 to 3 wherein the concentration of the H2 receptor antagonist ranges between 0.01 and 10% .

5. Compositions according to one or more of claims 1 to 4 wherein the concentration of the chitosan soluble derivative ranges between 0.001 and 10%.

6. Compositions according to one or more of claims 1 to 5 in the form of oral topical compositions.

7. Compositions according to claim 6 in the form of mouth-washes, gels, sprays, foams, emulsions, dentifrices.

8. Compositions according to one or more of claims 1 to 5 in the form of topical compositions for the application to the skin and vaginal and rectal mucous membranes.

9. Compositions according to claim 8 in the form of gel, creams, ointments, powders, patches, transdermal forms, periodontal coatings.

10. Compositions according to one or more of claims 1 to 9 further comprising other active ingredients selected from disinfectants of the oral cavity, steroidal or non steroidal antiinflammatories, wound healing agents, analgesics, antimicrobials, antihistaminics.

11. Compositions of claims 1-7 for the use in the prevention and therapy of odontostomatological pathologies **characterized by** inflammation and pain, in particular for the prevention and therapy of periodontium pathologies.

12. Compositions of claims 8-10 for use in the treatment of wounds or lesions of the skin or of rectal or vaginal mucous membranes.
